# EUROPEAN PATENT APPLICATION

(11) **EP 3 569 084 A1**
(43) Date of publication of application: **20.11.2019**
(21) Application number: 18172838.7
(22) Date of filing: 17.05.2018
(51) Int. Cl.: A42B 3/22, A61F 9/06

(54) **A WELDING HELMET WITH A STEPLESS ADJUSTABLE PROTECTIVE SHIELD STOP**

(71) Applicant: 3M Innovative Properties Company, Saint Paul, MN 55133-3427 (US)
(72) Inventor: Frejd, Jonas, 785 21 Gagnef (SE)
(74) Representative: Hohmann, Arno

(57) **Abstract**

A welding helmet (1) that has a protective shield (2) and a head suspension system (6). The protective shield and the head suspension system are pivotally connected to each other for swiveling the protective shield relative to the head suspension system about a pivot axis in opposite directions upward or downward between a protective position, in which the protective shield covers a wearer's face, and an upraised position, in which the protective shield uncovers the wearer's face. The welding helmet further has a stop mechanism (10) that restricts downward swiveling beyond the protective position. The stop mechanism is adjustable by a worm gear mechanism (11, 12) between a low position and a high position. In the low position the protective position of the protective shield is offset downward with respect to the high position.

## Description

### Field of the Invention

The invention relates to a welding helmet that comprises a protective shield and a head suspension system that can be swiveled between an upraised position and a protective position. In particular the invention relates to a welding helmet that allows for a stepless adjustment of the protective position of the protective shield between a low position and a high position.

### Background Art

Welding helmets are typically used in the mechanical and industrial art to protect welders from harmful irradiation emitted from the welding arc and from splashes, sparks and particles that may be ejected from welding area. Welding helmets typically can be suspended on the head of a wearer, so that the wearer has both hands available for welding and handling of workpieces.

Some welding helmets are furnished with an automatic darkening filter. An automatic darkening filter commonly has a switchable filter that automatically changes from a light-state to a dark-state in response to incident light generated by the welding arc. Thus, upon ignition of the welding arc the switchable filter automatically changes to the dark- state and protects the welder's eyes from the irradiation of the welding arc. Once the welding is interrupted or ended the switchable filter automatically changes to the light-state so that the user can see through the filter at normal light conditions.

Accordingly, there are welding helmets that stay in position on a wearer's head independent from the actual welding actions, for example during locating of the electrode toward the workpiece to be welded or during handling. There are welding helmets that have a protective shield that is used in a protective position and which can be lifted toward an upraised position so that the protective shield uncovers the wearer's face. This function is provided for example to allow the wearer to communicate or to handle a workpiece or welding equipment without the need to put off the welding helmet.

Some welding helmets can be fixed in the upraised position by tightening a screw, or the protective shield may be only movable at relatively high forces to avoid self-returning of the protective shield toward the protective position.

Although existing welding helmet provide for a variety of advantages there is still a need for a welding helmet that provides for easy handling and which helps maximizing the safety in the area of welding.

### Summary of the Invention

The invention relates to a welding helmet that comprises a protective shield and a head suspension system. The protective shield and a head suspension system are pivotally connected to each other. In particular the protective shield and the head suspension system are pivotally connected to each other for swiveling the protective shield relative to the head suspension system. The pivotal connection further provides for swiveling the protective shield relative to the head suspension system about a pivot axis in opposite directions upward or downward between a protective position, in which the protective shield covers a wearer's face, and an upraised position, in which the protective shield uncovers the wearer's face. The welding helmet further comprises a stop mechanism. The stop mechanism restricts downward swiveling of the protective shield beyond the protective position. The stop mechanism is adjustable by a worm gear mechanism between a low position and a high position. In the low position the protective position of the protective shield is offset downward with respect to the high position. Accordingly, in the high position the protective position of the protective shield is offset upward with respect to the low position.

The invention is advantageous in that it provides a welding helmet that has a protective shield which protective position can be adjusted in a stepless manner. This means that the protective position of the protective shield can be adjusted at any desired position between the low and the high position and not only at discrete predetermined positions. Further the adjustment can be accomplished by a single operation step, namely by turning a turning knob. Due to the natural self-locking character of a worm gear mechanism the adjusted position is automatically maintained.

In an embodiment the worm gear mechanism is arranged at the protective shield such that swiveling of the protective shield also causes swiveling of the worm gear mechanism. Preferably the worm gear mechanism comprises a worm shaft and a gear, which may be a full gear or a gear segment. The worm shaft and the gear are engaged with each other. The worm shaft and the gear are particularly engaged with each other such that the worm shaft can drive the gear. Further in the worm gear mechanism the worm shaft cannot be driven by the gear (self-locking character of a worm gear mechanism). The worm shaft preferably comprises a thread and a turning knob for turning the worm shaft.

In an embodiment the worm shaft is rotatably fixed by a holder. The fixation preferably enables for a rotation of the worm shaft about a worm shaft rotation axis. The fixation preferably is further such that the worm shaft is fixed in directions axially and radially of the worm shaft rotation axis.

In an embodiment the holder is in a fixed relationship with the protective shield. In particular the holder is preferably not rotatable or otherwise movable relative to the protective shield. For example, the holder may form a part of the protective shield. Further the gear is rotatably arranged relative to the protective shield. In particular the gear may be rotatably arranged relative to the protective shield for a rotation about the pivot axis by control of the worm gear mechanism or by control of the worm shaft. The fixation is preferably further such that the gear is fixed in directions axially and radially of the pivot axis. The stop mechanism preferably comprises a first engagement element. The first engagement element may protrude from the gear in a dimension parallel to the pivot axis and laterally offset from the pivot axis.

In a further embodiment the gear is in a fixed relationship with the protective shield. In particular the gear is preferably not rotatable or otherwise movable relative to the protective shield. The holder is preferably rotatably assembled to the protective shield. The assembly preferably provides for a rotation of the holder about the pivot axis by control of the worm gear mechanism or by control of the worm shaft. The stop mechanism preferably comprises a first engagement element that protrudes from the holder. The first engagement element may protrude from the holder in a dimension parallel to the pivot axis and laterally offset from the pivot axis.

In an embodiment the stop mechanism further comprises a second engagement element. The second engagement element is preferably provided for stopping the first engagement element at the protective position of the protective shield. The second engagement element is preferably (indirectly or directly) attached to the head suspension system. The protective shield may be displaceably attached to the head suspension system. In particular the protective shield may be displaceably attached to the head suspension system for a linear displacement in a dimension toward or away from the wearer's face. Thus, the wearer is enabled to adjust not only the protective position of the protective shield, but also the spacing between the wearer's face and the protective shield. The head suspension system may further consist of or comprise a headband.

In an embodiment the protective shield comprises a clear visor. The welding helmet may have a welding visor that is arranged pivotally relative to the clear visor for pivoting between a welding position in which the welding visor covers the clear visor and a non-welding position in which the welding visor uncovers the clear visor. Alternatively, the protective shield may comprise a welding visor instead of a clear visor. In this alternative no additional welding visor may be provided.

The welding visor may have an automatic darkening filter. The automatic darkening filter is preferably based on at least two liquid crystal cells. The liquid crystal cells are electrically switchable between a light-state and a dark-state. The two liquid crystal cells are preferably arranged optically in sequence. Each liquid crystal cell comprises two transparent substrates with a liquid crystal layer arranged between. Each substrate is provided with an alignment layer that is in contact with the liquid crystal layer. The alignment layers provide for a default uniform alignment of the liquid crystals. Further, the two liquid crystal cells preferably comprise three polarizers, one of which being arranged between the two liquid crystal cells and the other two being arranged on outer sides. The outer side polarizers may be arranged with their light polarizing orientation in the same or substantially the same direction, whereas the inner polarizer may be oriented with its light polarizing orientation 90 degrees or substantially 90 degrees relative to the outer polarizers.

In the light- state the transmittance of the automatic darkening filter may be within a range of about 1% to about 20%, in more particular within a range of about 5% to about 10%, whereas in the dark-state the transmittance of the automatic darkening filter may be within a range of about 0.0005% to about 0.1%.

In one embodiment the welding visor comprises a permanent optical filter (instead of an automatic darkening filter). Such an optical filter may have a permanent transmittance within a range of about 0.0005% to about 0.1%.

The welding visor may comprise further filters, in particular a UV filter (filtering ultraviolet light) and/or an IR (filtering infrared light).

In a further embodiment the welding helmet has at least one sensor for detecting light, as for example light emitted from the welding arc. The sensor and the automatic darkening filter are functionally interconnected so that light above a predetermined light intensity detected by the sensor causes the automatic darkening filter to switch to the dark-state and the absence or non-detection of light above the predetermined light intensity causes the automatic darkening filter to switch to the light-state.

In an embodiment the welding helmet has two or a plurality of sensors for detecting electromagnetic radiation. For example, one of the sensors may be adapted to sense at least visible light (which for the purpose of the present specification means electromagnetic radiation within a wavelength range from 400 nm to 700 nm), and a further sensor may be adapted to sense electromagnetic radiation outside visible light. One or more of the sensors may further be configured for sensing electromagnetic radiation within a range that covers at least a part of the visible light and at least a part outside the visible light (for example UV light).

Further, a plurality of the same or similar sensors may be arranged at different positions and/or at different sensing angles. This helps ensuring that at least one of the sensors detects the welding arc in case the other sensors are inadvertently obstructed.

In an embodiment the welding helmet further has a screw joint which pivotally connects at least the head suspension system and the protective shield. The screw joint may further pivotally connect the head suspension system, the protective shield and the welding visor. The screw joint preferably has a clamping wheel for fastening or loosening the screw joint and thus for or preventing or enabling, respectively, a swiveling of the head suspension system and the protective shield relative to each other.

### Brief Description of the Figures

- Fig. 1: is a side view of a welding helmet according to an embodiment of the invention;
- Fig. 2: is a further side view of the welding helmet shown in Fig. 1;
- Fig. 3: is a side view of the welding helmet shown in Fig. 1 at a different stage of operation;
- Fig. 4: is a view of a portion of a welding helmet having a stop mechanism according to an embodiment of the invention;
- Fig. 5.: is a view of the portion of the welding helmet shown in Fig. 4 at a different stage of operation;
- Fig. 6: is a cross-sectional view of the embodiment shown in Fig 4;
- Fig. 7: is a further cross-sectional view of a portion of the welding helmet shown in Fig. 4;
- Fig. 8: is a view of a portion of a further welding helmet having a stop mechanism according to an embodiment of the invention; and
- Fig. 9: is a view of another portion of the welding helmet shown in Fig. 8.

### Detailed Description of the Invention

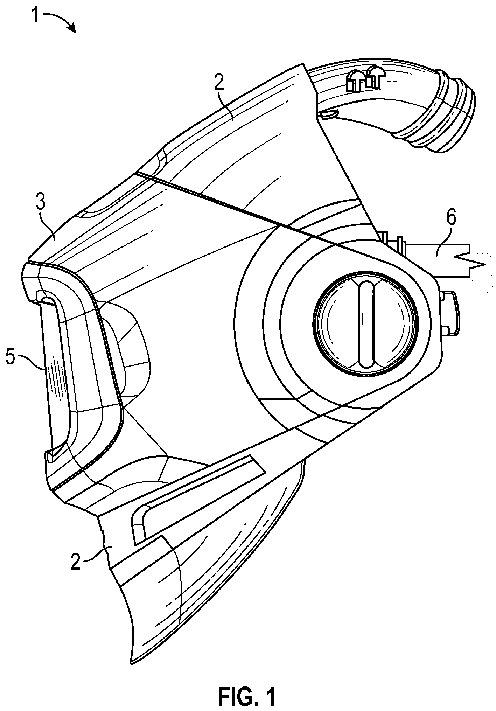

Fig. 1 shows a welding helmet 1 according to the invention. The welding helmet 1 has a protective shield 2 which is illustrated in a protective position that corresponds to a position in which the protective shield 2 covers a wearer's face. The welding helmet 1 further has a head suspension system 6 for supporting the welding helmet 1 on a wearer's head. The welding helmet 1 in the example further has a welding visor 3. The welding visor 3 is configured to filter those portions of light of a welding arc hat would be harmful for a wearer of the welding helmet 1 observing the welding arc through the welding visor 3.

The welding visor 3 therefore comprises an automatic darkening filter 5. The automatic darkening filter 5 allows a welder to safely observe the welding arc during welding. In the example the automatic darkening filter 5 is based on two liquid crystal cells. The liquid crystal cells are electrically switchable between a light-state and a dark-state. When switched in the dark-state, the automatic darkening filter 5 blocks a significant amount of light from being transmitted therethrough. This enables a user to observe a welding arc by seeing through the automatic darkening filter 5 without risking to be exposed to harmful light radiation from the welding arc. In the light-state the automatic darkening filter 5 permits a significant amount of light to be transmitted therethrough. Thus, the automatic darkening filter 5 in the light-state allows the user to see under ambient light conditions (in the absence of the welding arc). The two (or more) liquid crystal cells are arranged optically in sequence. This provides for multiplying the darkening effect (in particular in the dark-state) and thus a sufficient eye protection from light radiation.

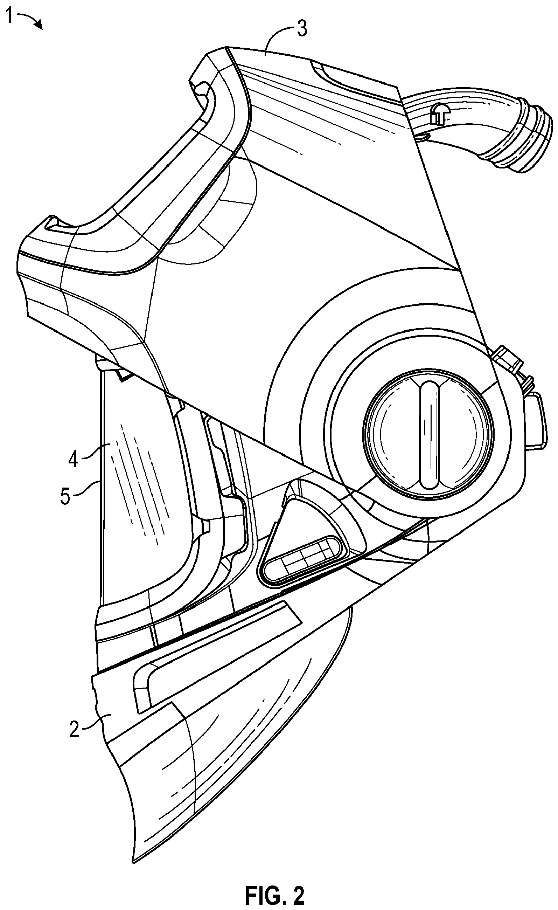

The welding visor 3 is illustrated in a welding position in which the welding visor 3 covers a see-through window (indicated as number 4 in Fig. 2) provided in the protective shield 2.

The welding visor 3 is however pivotable between the welding position (shown in Fig. 1) and a non-welding position (shown in Fig. 2).

Fig. 2 shows the welding helmet 1 with the welding visor 3 in the non-welding position in which the welding visor uncovers the see-through window 4. The protective shield 1 is still in the protective position. The protective shield 2 has a clear visor 5 that closes the see-through window 4 in the protective shield 2. Thus, a wearer's face is protected by the protective shield 2 and the clear visor 5 for example from particles ejected from a workplace the wearer faces toward. Although the clear visor 5 does not provide a sufficient protection against harmful light emitted from a welding arc, the clear visor 5 allows the wearer of the welding helmet 1 to see through the clear visor 5 at ambient light conditions. Therefore, in the protective position of the protective shield 2 with the welding visor 3 being in the non-welding position the welding helmet 1 may for example be used for protecting a wearer during grinding or similar works.

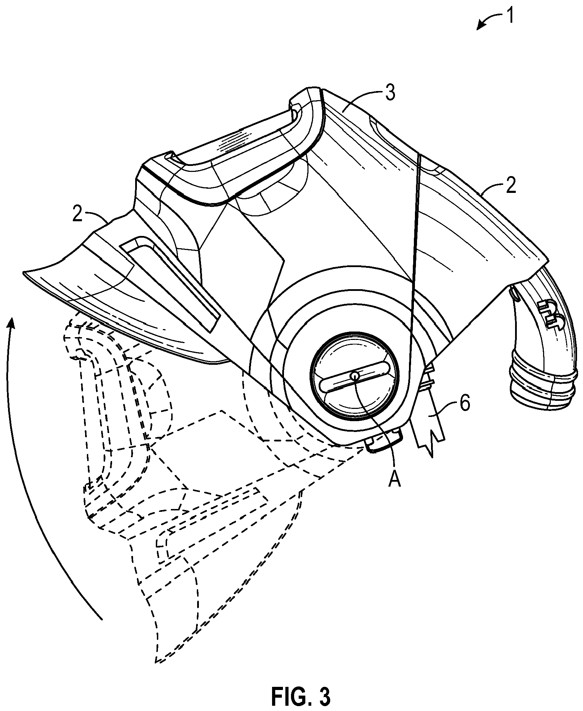

As illustrated in Fig. 3 the protective shield 2 of the welding helmet 1 further can be lifted upward or swiveled to an upraised position about a pivot axis A (extending perpendicular to the plane of the Figure through the point referred to as "A"). The swiveling of the protective shield 2 is provided relative to the head suspension system 6. Accordingly, the welding helmet 1 is configured such that the welding shield 2 can be swiveled on a wearer's head while the head suspension system remains in place. The swiveling of the protective shield 2 relative to the head suspension system is independent from the pivoting of the welding visor 3 relative to the protective shield 2. Thus, the position of the welding visor 3 relative to the protective shield 2 is not changed by changing the position of the protective shield 2 relative to the head suspension system. This allows a wearer of the welding helmet 1 to temporarily lift the protective shield 2 away from his or her face toward the upraised position and to continue using the welding helmet 1 with the protective shield 2 in the protective position after, without changing the position of the welding visor 3.

The welding helmet 1 is configured for adjusting the protective position between a low position and a high position. In the low position the protective shield is offset downward with respect to the high position and vice versa (in the high position the protective shield is offset upward with respect to the low position). This allows a wearer to adjust the welding helmet in the high position, for example, so that the protective shield does not touch the wearer's chest during works that require the wearer to look downwards. Alternatively, the wearer may adjust the welding helmet in the low position so that the wearer's face is protected during works in which the wearer looks upwards.

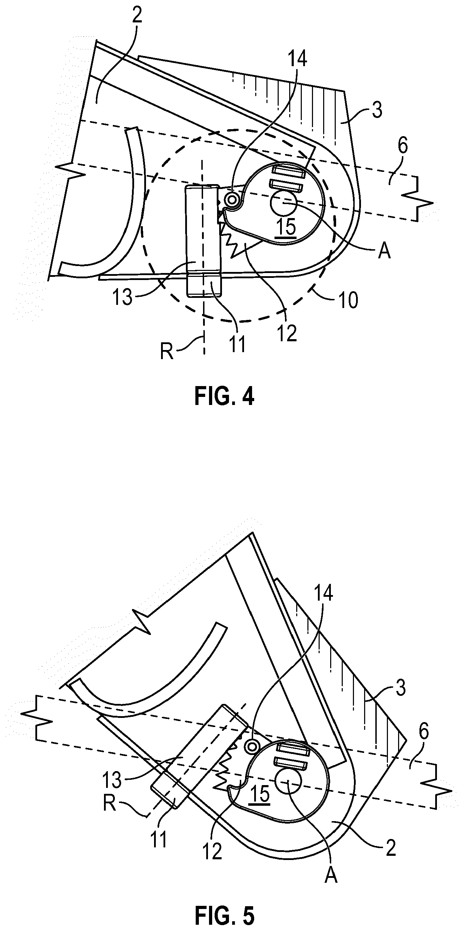

Fig. 4 shows a portion of the welding helmet 1. The welding helmet 1 comprises a stop mechanism 10 that restricts downward swiveling beyond the protective position. This means that in the protective position the protective shield cannot be swiveled further downward, although the protective position can be adjusted between the low and the high position. The stop mechanism 10 is adjustable by a worm gear mechanism that is part of the stop mechanism 10. The worm gear mechanism comprises a worm shaft 11 and a gear 12, which in the example is a gear segment. The worm shaft 11 is received within a holder 13. The worm shaft 11 is rotatable within the holder 13 about a worm shaft rotation axis R. However, the worm shaft 11 is otherwise fixed, in particular in directions axially and radially to the worm shaft rotation axis R. The holder 13 in the example is part of the protective shell 2. The skilled person will however recognize that the holder and the protective shell may be separate parts and assembled to each other. Accordingly, the holder 13 is in a fixed relationship, in particular fixed, with the protective shield 2, but rotatable about the shaft rotation axis R. The gear 12 further is rotatably arranged relative to the protective shield 2 for a rotation about the pivot axis A. The gear 12 is further otherwise fixed at the protective shield 2, in particular in directions axially and radially to the pivot axis A. The worm shaft 11 and the gear 12 are engaged with each other. Thus, the gear 12 can be rotated about the pivot axis A by control of the worm shaft 11. The gear 12 further comprises a first engagement element 14 that protrudes from the gear 12 in a dimension parallel to the pivot axis A and laterally offset from the pivot axis A. The stop mechanism 10 further comprises a second engagement element 15 for stopping the first engagement 14 element at the protective position (as shown) of the protective shield 2.

The second engagement element 15 is attached to the head suspension system 6. In the protective position of the protective shield 2 the first engagement element 14 and the second engagement element 15 are in engagement. The engagement is unidirectional, meaning that the first engagement element 14 and the second engagement element 15 stop each other when engaged but allow a movement away from each other.

Fig. 5 shows the same portion of the welding helmet 1 as Fig. 4 so that regarding any features and functions it is also referred to Fig. 4. As shown the protective shield 2 and the welding visor 3 are lifted to the upraised position (as also illustrated in Fig. 3). In the upraised position the first and second engagement element 14, 15 are offset or spaced from each other. The upraised position in the example is determined by the shape and configuration of the protective shield 2 and the welding visor 3. In particular, the welding visor 3 is stopped by the protective shield 2 in the upraised position against further swiveling upwards. The offset between the first and second engagement element 14, 15 in the upraised position defines the available swivel range from the upraised position to the protective position.

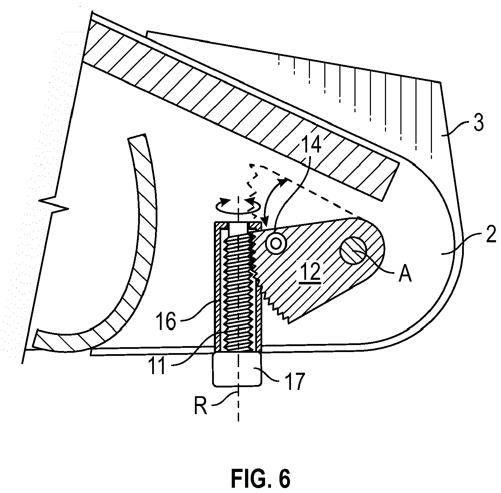

The protective position can be adjusted via the stop mechanism as illustrated in the cross-sectional view shown in Fig. 6. The worm shaft 11 comprises a thread 16 and a turning knob 17 for turning the worm shaft 11. Due to the engagement of the thread 16 of the worm shaft 11 with the gear 12, a rotation of the worm shaft 11 about the worm shaft axis R causes the gear 12 to rotate about the pivot axis A accordingly. Thereby the first engagement element 14 can be positioned between the high position (shown) and the low position (illustrated in dashed lines).

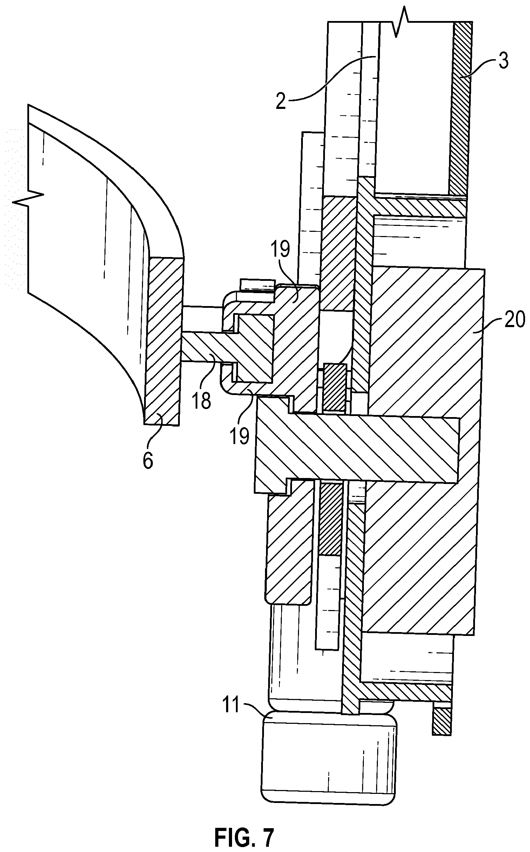

Fig. 7 shows the connection between the head suspension system 6, on the one hand, and the protective shield 2 and the welding visor 3, on the other hand. The head suspension system 6 in the example has slide bar 18. The slide bar has a T-shaped profile. A slider 19 is engaged on the slide bar 18 for a linear displacement along the slide bar 18. Thus, the protective shield 2 and the welding visor 3 are displaceably attached to the head suspension system 6 for a linear displacement relative to the head suspension system 6. The slider 19 comprises the first engagement element (not visible in this view). The slider is part of a screw joint that further has a clamping wheel 20. The screw joint extends through the protective shield 2 and the welding visor 3. The screw further suspends the protective shield 2 and the welding visor 3 for swiveling relative to the slider 19. The screw joint particularly can be fastened or loosened and thereby allows for clamping or unclamping, respectively, the protective shield 2 and the welding visor 3 relative to the slider 19.

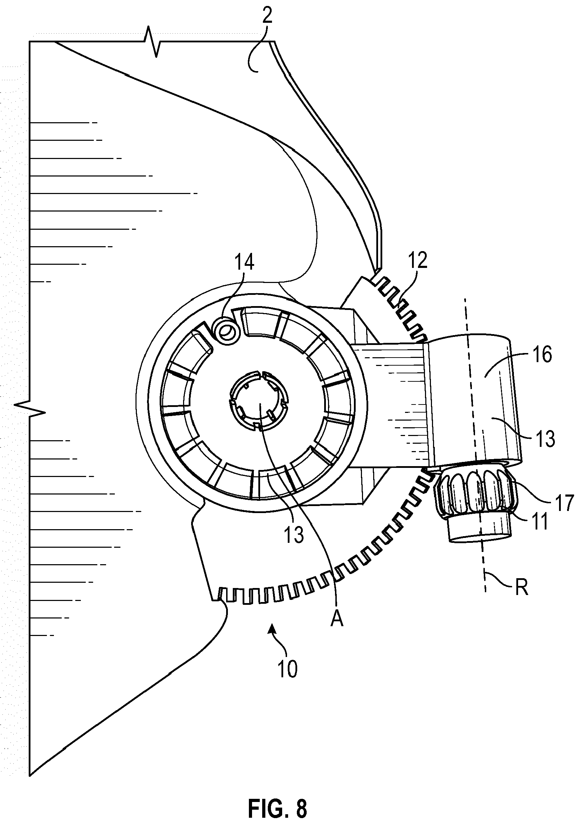

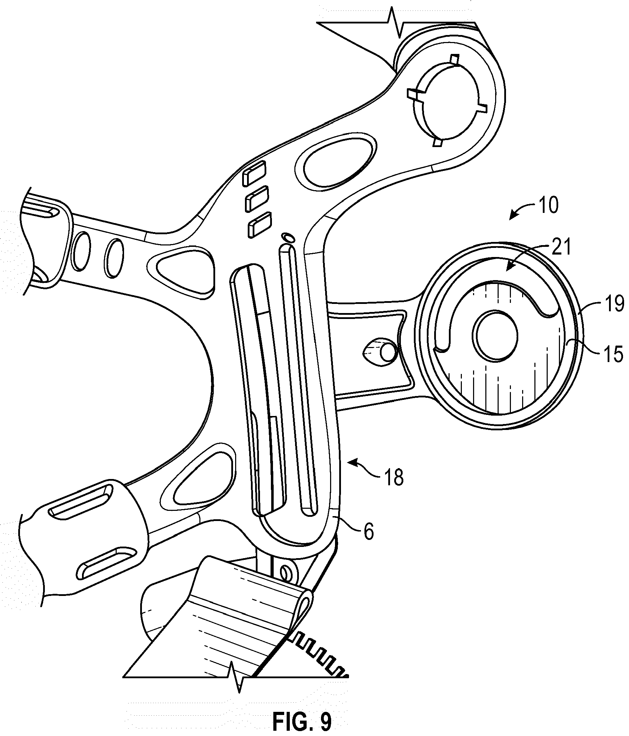

Figures 8 and 9 illustrate an alternative embodiment of the stop mechanism 10 of the invention. Fig. 8 shows a portion of the protective shield 2. The protective shield 2 comprises a gear 12. The gear 12 in the example is monolithically formed with the protective shield 2. In the Figure a holder 13 is rotatably arranged on the gear 12. At the assembled stage of the stop mechanism the holder 13 is rotatably arranged between the gear 12 and a slider 19 (shown in Fig. 9). The holder 13 has a first engagement element 14 for engaging into a C-shaped recess 21 within the sider 19. The recess 21 restricts a movement of the first engagement element 14 and thus in combination with the slider 19 forms a second engagement element 15. The stop mechanism 10 is adjustable by a worm gear mechanism that is part of the stop mechanism 10. The worm gear mechanism comprises the gear 12 and a worm shaft 11. The worm shaft 11 is received within the holder 13 and is engaged with the gear 12. The worm shaft 11 is rotatable within the holder 13 about a worm shaft rotation axis R. However, the worm shaft 11 is otherwise fixed, in particular in directions axially and radially to the worm shaft rotation axis R. In this embodiment the holder 13 is rotatably arranged relative to the protective shield 2 for a rotation about the pivot axis A. The holder 13 is further otherwise fixed relative to the protective shield 2, in particular in directions axially and radially to the pivot axis A. the first engagement element 14 protrudes from the holder 13 in a dimension parallel to the pivot axis A and laterally offset from the pivot axis A, and thus moves as the holder 13 moves. The holder 13 can be rotated about the pivot axis A by control of the worm shaft 11. Thereby the first engagement element 14 can be adjusted in position. The second engagement element 15 is part of the slider 19 and therefore in a fixed relationship to the head suspension system 6. In the protective position of the protective shield 2 the first engagement element 14 and the second engagement element 15 are in engagement. The protective position can be adjusted by means of the stop mechanism. The worm shaft 11 comprises a thread 16 (not visible) and a turning knob 17 for turning the worm shaft 11. Due to the engagement of the thread 16 of the worm shaft 11 with the gear 12, a rotation of the worm shaft 11 about the worm shaft axis R causes the holder 13 (and thus the first engagement element 14) to rotate about the pivot axis A accordingly. Thereby the first engagement element 14 can be positioned between the high position and the low position.

The slider 19 is engaged on a slide bar 18 of the head suspension system 6 for a linear displacement along the slide bar 18. Thus, the protective shield 2 and the welding visor 3 are displaceably attached to the head suspension system 6 for a linear displacement relative to the head suspension system 6.

## Claims

1. A welding helmet, comprising a protective shield and a head suspension system that are pivotally connected to each other for swiveling the protective shield relative to the head suspension system about a pivot axis in opposite directions upward or downward between a protective position, in which the protective shield covers a wearer's face, and an upraised position, in which the protective shield uncovers the wearer's face, the welding helmet further comprising a stop mechanism that restricts downward swiveling beyond the protective position, wherein the stop mechanism is adjustable by a worm gear mechanism between a low position and a high position, wherein in the low position the protective position of the protective shield is offset downward with respect to the high position.

2. The welding helmet of claim 1, wherein the worm gear mechanism is arranged at the protective shield such that swiveling of the protective shield also causes swiveling of the worm gear mechanism.

3. The welding helmet of claim 1 or 2, wherein the worm gear mechanism comprises a worm shaft and a gear that are engaged with each other, wherein the worm shaft comprises a thread and a turning knob for turning the worm shaft.

4. The welding helmet of claim 3, wherein the worm shaft is rotatably fixed by a holder for a rotation of the worm shaft about a worm shaft rotation axis.

5. The welding helmet of claim 4, wherein the holder is in a fixed relationship with the protective shield, and wherein the gear is rotatably arranged relative to the protective shield for a rotation about the pivot axis by control of the worm gear mechanism.

6. The welding helmet of claim 5, wherein the stop mechanism comprises a first engagement element that protrudes from the gear in a dimension parallel to the pivot axis and laterally offset from the pivot axis.

7. The welding helmet of claim 4, wherein the gear is in a fixed relationship with the protective shield, and wherein the holder is rotatably assembled to the protective shield for a rotation about the pivot axis by control of the worm gear mechanism.

8. The welding helmet of claim 7, wherein the stop mechanism comprises a first engagement element that protrudes from the holder in a dimension parallel to the pivot axis and laterally offset from the pivot axis.

9. The welding helmet of claim 6 or 8, wherein the stop mechanism further comprises a second engagement element for stopping the first engagement element at the protective position of the protective shield, wherein the second engagement element is attached to the head suspension system.

10. The welding helmet of any of the preceding claims, wherein the protective shield is displaceably attached to the head suspension system for a linear displacement in a dimension toward or away from the wearer's face.

11. The welding helmet of any of the preceding claims, wherein the head suspension system consists of or comprises a headband.

12. The welding helmet of any of the preceding claims, wherein the protective shield comprises a clear visor, and wherein the welding helmet has a welding visor that is arranged pivotally relative to the clear visor for pivoting between a welding position in which the welding visor covers the clear visor and a non-welding position in which the welding visor uncovers the clear visor.

13. The welding helmet of claim 12, wherein the welding visor has an automatic darkening filter.

14. The welding helmet of claim 12 or 13, further having a screw joint which pivotally connects at least the head suspension system and the protective shield.

15. The welding helmet of claim 14, wherein the screw joint has a clamping wheel for fastening or loosening the screw joint and thus for or preventing or enabling, respectively, a swiveling of the head suspension system and the protective shield relative to each other.
